(19) Europäisches Patentamt
European Patent Office
Office européen des brevets

(11) **EP 2 378 272 B1**

(12) **EUROPEAN PATENT SPECIFICATION**

(45) Date of publication and mention
of the grant of the patent:
**15.05.2013 Bulletin 2013/20**

(51) Int Cl.:
*A61B 5/00* *(2006.01)*      *A61B 5/06* *(2006.01)*
*A61B 8/08* *(2006.01)*      *G01N 21/49* *(2006.01)*
*G01N 21/17* *(2006.01)*      *G01N 29/24* *(2006.01)*

(21) Application number: **10786103.1**

(22) Date of filing: **02.06.2010**

(86) International application number:
**PCT/JP2010/059339**

(87) International publication number:
**WO 2010/143572 (16.12.2010 Gazette 2010/50)**

(54) **SUBJECT INFORMATION ANALYSIS DEVICE AND SUBJECT INFORMATION ANALYSIS METHOD**

VORRICHTUNG ZUR ANALYSE VON SUBJEKTINFORMATIONEN SOWIE VERFAHREN ZUR ANALYSE VON SUBJEKTINFORMATIONEN

DISPOSITIF ET MÉTHODE D'ANALYSE D'INFORMATIONS SUR UN SUJET

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **12.06.2009 JP 2009141414**

(43) Date of publication of application:
**19.10.2011 Bulletin 2011/42**

(73) Proprietors:
• **Olympus Corporation**
**Shibuya-ku**
**Tokyo 151-0072 (JP)**
• **Olympus Medical Systems Corp.**
**Tokyo 151-0072 (JP)**

(72) Inventors:
• **INNAMI, Takeharu**
**Tokyo 151-0072 (JP)**
• **IGARASHI, Makoto**
**Tokyo 151-0072 (JP)**

(74) Representative: **Schicker, Silvia**
**Wuesthoff & Wuesthoff**
**Patent- und Rechtsanwälte**
**Schweigerstrasse 2**
**81541 München (DE)**

(56) References cited:
**EP-A1- 1 810 610**      **WO-A2-03/050249**
**JP-A- 2000 088 743**      **JP-A- 2000 088 743**
**JP-A- 2005 224 399**      **JP-A- 2008 170 363**
**JP-A- 2008 170 363**      **JP-T- 2006 520 893**
**US-A- 6 041 248**

• **MAHAN G D ET AL: "ULTRASONIC TAGGING OF LIGHT: THEORY", PROCEEDINGS OF THE NATIONAL ACADEMY OF SCIENCES, NATIONAL ACADEMY OF SCIENCES, vol. 95, 1 January 1998 (1998-01-01), pages 14015-14019, XP001028174, ISSN: 0027-8424, DOI: 10.1073/PNAS.95.24.14015**

**Description**

Technical Field

The present invention relates to a subject information analyzing apparatus and a subject information analyzing method that receives scattered light from a region to be examined of a subject irradiated with ultrasound and make an analysis such as generating feature information for discriminating tissue properties.

Background Art

In recent years, various techniques such as optical CT, optical coherence tomography (hereinafter referred to as "OCT"), optical acoustic tomography have been proposed as techniques for realizing optical tomographic imaging of a living body.

For example, Japanese Patent Application Laid-Open Publication No. 2008-170363 discloses an analyzing apparatus and method for detecting differences in a scattering coefficient as feature information between normal tissue and cancerous tissue by radiating ultrasound and light onto the living body as a subject and detecting an amount of Doppler shift of scattered light caused by interaction between light and ultrasound in mucous membrane in the living body.

In the conventional example of the above described publication, ultrasound modulated light as modulated light through ultrasound vibration from the subject is detected using an optical detector, the frequency and wavelength of the detected signal are analyzed, the amount of Doppler shift is obtained and those values are converted to scattering coefficients. Therefore, in order to depict a slight difference between the scattering coefficients, it is rendered necessary to analyze a slight change of the amount of Doppler shift as frequency resolution.

In the above conventional example, in order to enhance the frequency resolution, it is necessary to increase a sampling rate of the signal detection. In this case, it takes considerable time for the signal processing for depicting the slight difference between the scattering coefficients. Further, in the case of increasing the sampling rate, a high precise signal processing at higher speed (hither frequency) is required and therefore an apparatus of higher price is necessitated.

In view of the above, it has been strongly desired to provide a subject information analyzing apparatus and a method for the apparatus capable of generating feature information of constituent tissues constituting the region to be examined without need of enhancing the frequency resolution and also performing analysis such as discrimination of properties of the tissue as to whether it is normal tissue or lesion tissue.

In this case, as the feature information of constituent tissues, information suitable for diagnosing or discriminating whether the constituent tissues are normal tissues or lesion tissues, etc., or information regarding a composition ratio of the constituent tissues to be used effectively in the diagnosis is desirable.

The present invention has been made in view of the above points and an object of the present invention is to provide a subject information analyzing apparatus and a subject information analyzing method which enable to generate feature information on a composition ratio of the constituent tissues of the region to be examined of the subject without need of a high frequency resolution with respect to the frequency-modulated light.

Document EP 1 810 610 A1 discloses a subject information analysing apparatus, comprising a light receiving means that receives frequency-modulated light as scattered light corresponding to irradiating light of a second frequency from a region to be examined in a subject irradiated with ultrasound of a first frequency, an information extracting means that extracts information, and a feature information generating means. In this context, also documents US 6,041,248 A, WO 03/050249 A2, JP 2-000088743 A, JP 2008-170363 A, and document MAHAN G D ET AL: "ultrasonic tagging of light: theory", proceedings of the national academy of sciences, vol. 95, 1 January 1998, pages 14015-14019, XP001028174 should be mentioned.

Disclosure of Invention

Means for Solving the Problem

A subject information analyzing apparatus according to an aspect of the present invention according to claim 1 is provided.

Furthermore, a subject information analyzing method according to an aspect of the present invention according to claim 9 is provided.

Brief Description of the Drawings

Fig. 1 is a block diagram illustrating a configuration of a biological information analyzing apparatus according to a first embodiment of the present invention;

Fig. 2 is a diagram illustrating a relationship between a volume fraction and nuclei/cytoplasm ratio (N/C ratio);

Fig. 3 is a timing chart for illustrating operation of the first embodiment;

Fig. 4 is a flowchart illustrating a typical example of a processing procedure of the first embodiment;

Fig. 5 is a block diagram illustrating a configuration of a biological information analyzing apparatus according to a second embodiment of the present invention;

Fig. 6 is a flowchart illustrating a typical example of a processing procedure of the second embodiment; and

Fig. 7 is a block diagram illustrating a configuration of a biological information analyzing apparatus according to a modification example of the second embodiment.

Best Mode for Carrying Out the Invention

Hereinafter, embodiments of the present invention will be described with reference to the accompanying drawings.

(First embodiment)

Before describing embodiments of the present invention, the principles thereof will be described.

When ultrasound is impinged onto a region to be examined in a subject, vibration is provoked on particles (cell nuclei making up a biological tissue in embodiments which will be described later) making up the region to be examined in the subject according to propagation of ultrasound. A vibration speed of particles $v(t)$ at time t can be expressed by equation (1).

$$v(t) = v_{max} \cos(\omega_{us} t) \qquad (1)$$

Here, $v_{max}$ is a maximum value of vibration speed of particles, $\omega_{us}$ is an angular frequency of ultrasound. On the other hand, when light is impinged into the subject, the light is scattered by the above described particles. Furthermore, the incident light is affected by a Doppler effect through vibration of particles, the angular frequency $\omega(t)$ of the scattered light varies compared to the incident light and this angular frequency $\omega(t)$ can be expressed by equation (2).

$$\omega(t) = \omega_o \cdot c/(c - v(t) \cdot \cos\alpha) = \omega_o \cdot c/(c - v_{max} \cos(\omega_{us} t) \cdot \cos\alpha) \approx \omega_o [1 + v_{max} \cos(\omega_{us} t) \cdot \cos\alpha/c] \qquad (2)$$

Here, $\omega_o$ is an angular frequency of incident light, c is the velocity of light in the subject, $\alpha$ is an angle formed between the advancing directions of light and ultrasound and an approximation of $v_{max} \ll c$ is adopted in the last part of equation (2).

Intensity I of the scattered light affected by the Doppler effect is frequency-modulated as shown in equation (3).

$$I = I_o \cos[\theta(t)] = I_o \cos[\int \omega(t) dt] = I_o \cos[\omega_o t + (\omega_o/\omega_{us}) \cdot (v_{max} \cdot \cos\alpha/c) \cdot \sin(\omega_{us} t) + \phi_o]$$
$$= I_o \cos[\omega_o t + m \cdot \cos(\omega_{us} t) + \phi_1] = I_o \Sigma_L J_L(m) \cos[(\omega_o + L\omega_{us})t + L\pi/2 + \phi_1] \qquad (3)$$

Here, $I_o$ is amplitude of intensity of light, $\theta(t)$ is phase of light, $\phi_o$ and $\phi_1$ are initial phases, $J_L(m)$ is a first kind Bessel function, L is an integer, $\Sigma_L$ represents obtaining a sum total of the right side of $\Sigma$ when L varies from $-\infty$ to $+\infty$.

Furthermore, m is a degree of modulation and is expressed by equation (4).

$$m = (\omega_o/\omega_{us}) \cdot (v_{max} \cdot \cos\alpha/c) = \cos\alpha \cdot v_{max}/(f_{us} \cdot \lambda_o) \qquad (4)$$

Here, $f_{us}$ ($= \omega_{us}/2\pi$) is a frequency of ultrasound, $\lambda_o$ is a wavelength of incident light.

From equation (3), the spectrum of frequency-modulated scattered light has an angular frequency $\omega_o$ of incident light and an angular frequency shifted from the angular frequency $\omega_o$ by an angular frequency $L\omega_{us}$ which is an integer multiple of an angular frequency $\omega_{us}$ of ultrasound and it is possible to calculate a degree of modulation m from intensity information of a plurality of different spectra.

In this case, in principle, the degree of modulation m can be calculated if the frequency resolution is higher than the order of the angular frequency $\omega_{us}$ of ultrasound. Therefore, the present embodiment allows the degree of modulation m to be calculated without requiring high frequency resolution. As will be described later, a frequency $f_{us}$ of ultrasound within a frequency range of several MHz to several tens of MHz is typically widely used.

Furthermore, a maximum value $V_{max}$ of vibration speed of particles in equation (4) can be calculated by calculating the degree of modulation m.

On the other hand, the maximum value $v_{max}$ of vibration speed can be expressed by equation (5) using density $\rho$, velocity of sound $V_{us}$ and ultrasound intensity $I_{us}$.

$$v_{max} = (2I_{us}/(\rho V_{us}))^{1/2} \qquad (5)$$

Furthermore, when Urick's model for a suspension is assumed, the velocity of sound $V_{us}$ in the suspension can be expressed by equation (6).

$$(V_{us,v}/V_{us})^2 = [1 + X_p(K_p^{-1} - K_v^{-1})/K_v^{-1}][1 + X_p(\rho_p - \rho_v)/\rho_v] \qquad (6)$$

Here, $V_{us,v}$ is the velocity of sound of the medium, $X_p$ is a volume fraction as the composition ratio occupied by particles in the medium (or also referred to as "volume occupation rate" or simply "occupation rate"), $\rho$ is a density when particles of volume fraction $X_p$ exist in the medium, K is a modulus of volume elasticity and distinguished by adding subscripts p and v to the particles and the medium respectively.

As will be described later, particles and the medium are assumed to be made up of cell nuclei and cytoplasm making up a cell as constituent tissues of the region to be examined in a living body. Therefore, the above described volume fraction $X_p$ is information on the composition ratio corresponding to the ratio of volume occupied by cell nuclei among the volume of cells in the following embodiment.

Furthermore, as shown in equation (7) below, the density $\rho$ is expressed as a value obtained by adding a value resulting from multiplying the volume fraction $X_p$ of particles by a density $\rho_p$ of particles a value obtained by multiplying a value resulting from subtracting the volume fraction $X_P$ of particles from 1 by the density $\rho_v$ of the medium.

$$\rho = X_p\rho_p + (1 - X_p)\rho_v \qquad (7)$$

(In order to describe a variation of bloated cell nuclei in lesioned tissue which will be described later) when the radius of particles changes from r to r', the volume fraction occupied by particles changes from $X_p$ expressed by equation (8) to $X'_p$ expressed by equation (9).

However, such approximation is made that there is no variation in physical property values of particles and the surrounding medium.

$$X_p = N_o \cdot 4\pi r^3/(3 \cdot V) \qquad (8)$$

$$X'_p = N_o' \cdot 4\pi r'^3/(3 \cdot V) \qquad (9)$$

Here, V denotes the volume of the ultrasound irradiated region (local region of the region to be examined 18) where ultrasound is made to converge and locally irradiated and No and $N_o'$ denote the number of particles before and after a variation of the radius of particles in the ultrasound irradiated region V.

When the radius of particles varies from r to r', the velocity of sound changes from $V_{us}$ to $V'_{us}$ and the density changes from $\rho$ to $\rho'$. Furthermore, according to equation (5), the maximum value of vibration speed of particles changes

from $v_{max}$ to $v'_{max}$ due to difference in the radius of particles.

Therefore, according to equation (4), the degree of modulation m differs depending on differences in the radius of particles, and therefore by measuring $J_L(m)$ in a plurality of different Ls, it is possible to calculate the degree of modulation m and further calculate the volume fraction of particles(or as particulate component demonstrating particulate characteristics) making up the subject (ultrasound irradiated region therein) from the calculated degree of modulation m.

As shown in equation (13), which will be described later, the degree of modulation m can be calculated by measuring a plurality of L values, for example, intensity of $J_L(m)$ in two integer values of L=0, 1.

As described so far, by detecting frequency-modulated light (that is, ultrasound modulated light) using ultrasound and calculating the degree of modulation m thereof, it is possible to image a volume fraction $X_p$ of particulate components in a subject or a distribution of the volume fraction variation $\Delta X_p$.

When a normal tissue in a living body is compared with a cancerous tissue as a lesioned tissue, hypertrophy of cells and cell nuclei (also simply referred to as "nuclei") or the like occurs due to the cancerous tissue. In cancerous cells in particular, compared to normal cells, it is generally known that the ratio of increase of nuclei is higher than that of cytoplasm, in other words, the nuclei/cytoplasm ratio (abbreviated as "N/C ratio") increases.

Thus, the value of N/C ratio as feature information of composition ratio of nuclei and cytoplasm making up cells in cell tissues as constituent tissues of the region to be examined (or region to be diagnosed) constitutes important information to discriminate or diagnose properties as to whether its cell tissue is a normal tissue or a lesioned tissue. Therefore, in the present embodiment, the N/C ratio will be calculated as follows.

Assuming the N/C ratios between the normal tissue and the cancerous tissue are $(N/C)_n$ and $(N/C)_d$ respectively, these can be expressed as equation (10) and equation (11).

$$(N/C)_n = (3\pi^{1/2} X_p/4)^{2/3} \qquad (10)$$

$$(N/C)_d = (3\pi^{1/2} X'_p/4)^{2/3} \qquad (11)$$

Here, assuming a cube-shaped cell, suppose the length of one side of the cell is d (its volume $u_o = d^3$), the radius of the nucleus in the cell is r (volume $u_1 = 4\pi r^3/3$), the number of cells in the ultrasound irradiated region, that is, the number of nuclei is $N = V/u_o$, and volume fraction is $X_p = u_1/u_o$.

Then, since the N/C ratio becomes $N/C = \pi r^2/d^2$, this can be calculated as shown in equations (10) and (11) above.

As described so far, the degree of modulation m of ultrasound modulated light takes different values between the normal tissue and the cancerous tissue as degree of modulations $m_n$ and $m_d$ respectively.

Therefore, by calculating the degree of modulation m of the ultrasound modulated light, $(N/C)_n$ and $(N/C)_d$ can be calculated and imaged as feature information of the constituent tissues making up the region to be examined in the subject. Instead of $(N/C)_n$ and $(N/C)_d$, closely related volume fraction $X_p$ may also be calculated and imaged as feature information.

Furthermore, when the degree of modulation m is calculated for calculating feature information, information on spectral intensity of a specific frequency of ultrasound modulated light needs to be measured.

As is clear from the description above, the distance between, for example, fundamental and second harmonic in a spectrum of ultrasound modulated light is a frequency $f_{us}$ of ultrasound irradiated and calculation of the degree of modulation m does not require high frequency resolution. In principle, the degree of modulation m can be calculated if the frequency resolution is higher than the order of frequency $f_{us}$ of ultrasound.

Therefore, the apparatus and method according to the present embodiment have an advantage of solving problems in the prior arts as described above.

Next, influences of absorption of light by a subject (suppose its absorption coefficient is $\mu_a$) on the detection of scattered light will be described. When the subject absorbs light, the intensity of scattered light can be expressed as equation (12) which is modified equation (3), which does not take the absorption into account.

$$I = I_o \exp(-\mu_a D) \Sigma_L J_L(m) \cos[(\omega_o + L\omega_{us})t + L\pi/2 + \varphi_1] \qquad (12)$$

Here, D is a distance that the ultrasound modulated light propagates in the subject. According to equation (12), the absorption affects only the amplitude portion of intensity.

For this reason, in the spectrum component on the right side of equation (12), by comparing a Oth-order component $I_{f=0}$ when, for example, L=0 and L=1 with a first-order component $I_{f=fus}$ as shown in equation (13), it is possible to remove influences of the absorption and calculate the degree of modulation m.

$$I_{f=fus}/I_{f=0}=I_o exp(-\mu_a D)J_1(m)/I_o exp(-\mu_a D)J_o(m)$$

$$= J_1(m)/J_o(m) \approx m/2 \qquad (13)$$

Here, the rightmost side becomes approximation that holds when m<<1.

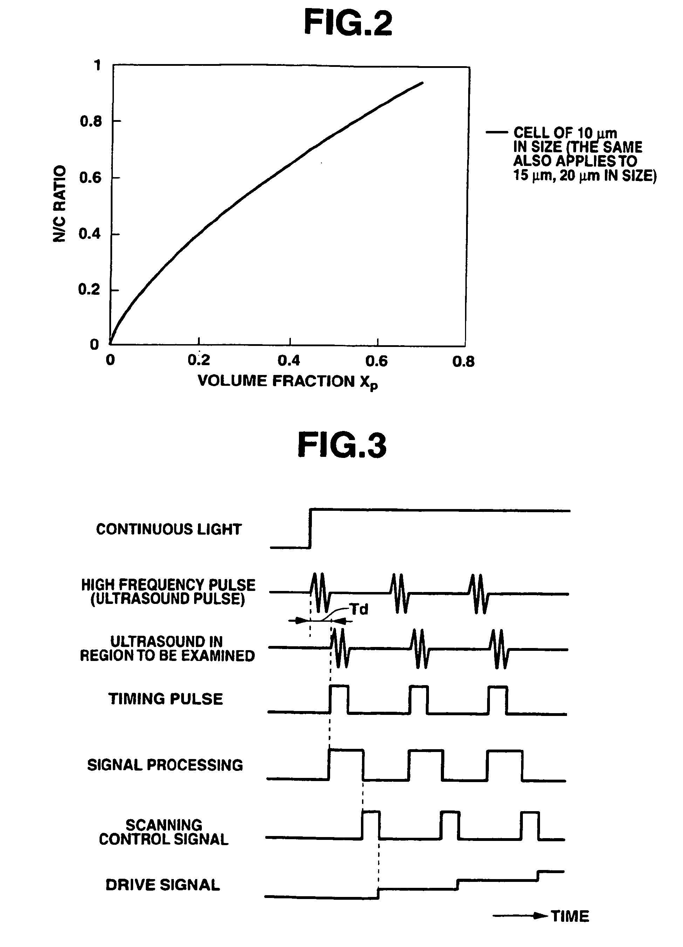

Furthermore, Fig. 2 is a graph illustrating equation (10). Here, with the horizontal axis showing the volume fraction $X_p$ and the vertical axis showing the N/C ratio, Fig. 2 describes a relational expression with respect to a plurality of different d's (length of one side of the cell) in a graph form.

To be more specific, when a cell having a size of d=10 $\mu$m, 15 $\mu$m and 20 $\mu$m is assumed, the graph shows that the N/C ratio can be uniquely calculated from the volume fraction $X_p$ regardless of the size (curves overlap).

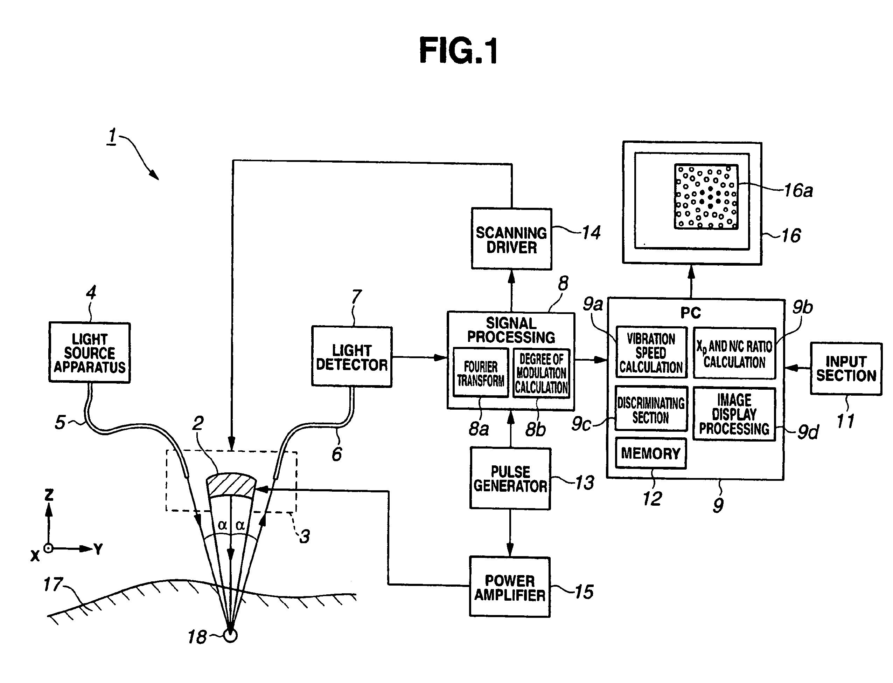

Next, the configuration of a biological information analyzing apparatus 1 as a first embodiment of the subject information analyzing apparatus of the present invention will be described with reference to Fig. 1.

The biological information analyzing apparatus 1 includes an ultrasound transducer 2 as ultrasound irradiating means (ultrasound generating means).

The ultrasound transducer 2 radiates (generates) ultrasound, for example, of a first frequency (to be more specific, aforementioned frequency $f_{us}$) so as to reach a region to be examined (or region to be analyzed) 18 in a living body 17 (for example, in the body cavity) as a subject along a predetermined ultrasound transmission axis.

The ultrasound transducer 2 is mounted in a scanning section 3 as scanning means and scans (moves over) the region to be examined 18 on which the scanning section 3 focuses and radiates ultrasound. The scanning section 3 is mounted with a piezoelectric element that scans, for example, in two directions on, for example, a scanning stand on which the ultrasound transducer 2 is mounted.

Nuclei as mainly particles or particulate components in living body constituent tissues of the region to be examined 18 are locally vibrated by ultrasound through irradiation with focused ultrasound as shown in equation (1).

Furthermore, the biological information analyzing apparatus 1 has a light source apparatus 4 that generates light of a second frequency (to be more specific frequency $f_0=\omega_0/(2\pi)$). The light source apparatus 4 is made up of, for example, a laser diode which is suitable for generating light of, for example, a single frequency. In this case, the light emitted from the light source apparatus 4 is laser light.

The light generated by the light source apparatus 4 is made to impinge on one end of an optical fiber 5 as a light guiding section. The optical fiber 5 guides the incident light and emits the light from its distal end portion.

The distal end, portion is also arranged, for example, at a position neighboring one side of the ultrasound transducer 2 in the scanning section 3. An angle formed between the advancing direction of light emitted from the optical fiber 5 and the advancing direction of ultrasound is $\alpha$.

The light emitted from the distal end portion of the optical fiber 5 is radiated onto the region to be examined 18 as the local region in the living body 17. The distal end portion of the optical fiber 5 forms light irradiating means for radiating light of a predetermined frequency onto a region to be examined 18 in the living body 17. The region to be examined 18 is scanned as the scanning section 3 moves.

The ultrasound vibrating constituent tissues (mainly nuclei) in the local region of the region to be examined 18 function as a light scattering substance that scatters the irradiated (impinged) light. The scattered light by the light scattering substance becomes light whose frequency is modulated from a frequency $f_0$ of light before scattering due to a Doppler effect caused by ultrasound vibration.

Furthermore, a distal end portion of an optical fiber 6 is arranged neighboring the other side of the ultrasound transducer 2 in the scanning section 3.

Scattered light of the light radiated onto the region to be examined 18 is impinged on the distal end portion of the optical fiber 6. The frequency-modulated light impinged on the optical fiber 6 as scattered light is expressed by equation (2) or equation (3). The angle of incidence of the light impinged on the optical fiber 6 is also set to $\alpha$.

The optical fiber 6 guides the light impinged on the distal end portion to a light detector 7 made up of a photodiode or the like. The light detector 7 receives (detects) frequency-modulated light as scattered light and outputs a detected signal as an electric signal photoelectrically converted from the received light to a signal processing apparatus 8.

That is, the light detector 7 forms light receiving means for receiving frequency-modulated light as scattered light from the region to be examined 18. Scattered light may also be directly received by the light detector 7 without using the optical fiber 6. In this case, the light detector 7 may be attached to the scanning section 3.

The signal processing apparatus 8 includes a Fourier transform circuit 8a that amplifies the detected signal of the scattered light, A/D converts and Fourier transforms the signal.

The signal processing apparatus 8 separates the signal into spectral intensities for extracting intensity information of a specific frequency through Fourier transform by the Fourier transform circuit 8a.

The intensity I of the scattered light is separated (extracted) into intensity information of specific frequencies having intensities of $I_0J_L(m)$ respectively at frequency positions shifted by an integer multiple (L=0, $\pm1$, $\pm2$,...) of the frequency $f_{us}$ of ultrasound from the position of frequency $f_0$ of light on the frequency axis as shown on the right side of equation (3) through Fourier transform (but not shifted if L=0). When influences of absorption are taken into account, common factors are added.

That is, the Fourier transform circuit 8a has a function of information extracting means for electrically extracting information of a specific frequency shifted by an integer multiple of frequency $f_{us}$ of ultrasound. In a second embodiment, information extracting means is formed for optically extracting information of a specific frequency shifted by an integer multiple of frequency $f_{us}$ of ultrasound using optical interference.

The information extracting means for extracting information on a specific frequency or information correlated with this can also be regarded as at least one of the Fourier transform circuit 8a, a degree of modulation calculation section 8b that calculates the degree of modulation m (which will be described later) and a vibration speed calculation section 9a that calculates a maximum value $v_{max}$ of vibration speed (which correlates with the degree of modulation m).

The signal processing apparatus 8 has a function of the degree of modulation calculation section 8b that calculates the degree of modulation m (as information on the specific frequency) from the information on intensity of the specific frequency.

The degree of modulation calculation section 8b calculates the degree of modulation m by calculating a ratio of the Oth-order component $I_{f=0}$ to the first-order component $I_{f=fus}$ corresponding to spectral intensity of L=0 and L=1 in intensity I of scattered light as shown, for example, in equation (13).

The embodiment is not limited to the case where the degree of modulation m is calculated from the ratio of the Oth-order component $I_{f=0}$ to the first-order component $I_{f=fus}$ of L=0 and L=1, but the degree of modulation m may also be calculated using another value ofL (e.g., L=2, 3). At least two different L values may be preferably used to calculate the degree of modulation m by removing influences of absorption.

The signal processing apparatus 8 outputs information on the calculated degree of modulation m to the personal computer (abbreviated as "PC") 9. The embodiment is not limited to the case where the signal processing apparatus 8 calculates the degree of modulation m as information on the specific frequency, but such a configuration may also be adopted that the PC 9 calculates the degree of modulation m. That is, the PC 9 may be configured to have the function of the signal processing apparatus 8.

The PC 9 calculates a maximum value $v_{max}$ of vibration speed of nuclei corresponding to particles in equation (4) using information on the degree of modulation m, an angle $\alpha$ formed between the advancing directions of light and ultrasound in equation (1) inputted by an operator from the input section 11, wavelength $\lambda_0$ of light, frequency $f_{us}$ of ultrasound or the like. That is, the PC 9 has a function of a vibration speed calculation section 9a that calculates a maximum value $v_{max}$ of vibration speed.

The PC 9 incorporates, for example, a memory 12 and stores information such as angle $\alpha$, wavelength $\lambda_0$ of light, frequency $f_{us}$ of ultrasound inputted from the input section 11 or the like. The PC 9 then calculates a maximum value $v_{max}$ of the vibration speed from the degree of modulation m inputted from the signal processing apparatus 8 with reference to the information stored in the memory 12. The PC also stores the inputted degree of modulation m, the calculated maximum value $v_{max}$ of the vibration speed in the memory 12.

Furthermore, the PC 9 performs fitting on the calculated maximum value $v_{max}$ of the vibration speed using the relationship in equation (5), using equation (6) and equation (7) and using the volume fraction $X_p$ occupied by cell nuclei as a parameter.

Through the fitting, the PC 9 calculates the volume fraction $X_p$ of nuclei as feature information on the composition ratio of cell tissues as the constituent tissues of the region to be examined 18 corresponding to the maximum value $v_{max}$ of the vibration speed obtained by equation (5).

Furthermore, after calculating the volume fraction $X_p$, the PC 9 calculates an N/C ratio as feature information of the composition ratio between nuclei and cytoplasm using the calculated value of the volume fraction $X_p$.

That is, the PC 9 includes a volume fraction and N/C ratio calculation section (abbreviated as $X_p$ and N/C ratio calculation in Fig. 1) 9b that calculates volume fraction $X_p$ and N/C ratio as feature information. The volume fraction and N/C ratio calculation section 9b has a function of feature information generating means for generating feature information on a composition ratio of constituent tissues of the region to be examined 18.

Although the volume fraction and N/C ratio calculation section 9b in the present embodiment is shown in the configuration so as to calculate the volume fraction $X_p$ and N/C ratio, the volume fraction and N/C ratio calculation section 9b is not limited to this configuration but may also be configured so as to include only a volume fraction calculation section that calculates a volume fraction or N/C ratio calculation section that calculates an N/C ratio.

Furthermore, as shown in Fig. 1, the signal processing apparatus 8 outputs a scanning control signal for causing a scanning driver 14 to scan in synchronization with a high frequency pulse (shifted by a predetermined time) generated from a pulse generator 13 that generates an ultrasound drive signal for generating, for example, a pulsed ultrasound.

The pulse generator 13 generates a high frequency pulse for generating pulsed ultrasound from the ultrasound transducer 2 and this high frequency pulse is amplified by a power amplifier 15 and applied to the ultrasound transducer 2. The ultrasound transducer 2 emits pulsed ultrasound according to the applied high frequency pulses.

The high frequency pulse is a pulse including a sine wave on the order of, for example, several cycles that causes the ultrasound transducer 2 to ultrasound vibrate at a frequency $f_{us}$ as shown in Fig. 3. The ultrasound transducer 2 ultrasound vibrates at a frequency $f_{us}$ of sine wave and emits the pulsed ultrasound along the ultrasound transmission axis from the concave output surface.

The frequency $f_{us}$ of ultrasound in this case is, for example, on the order of several MHz to several tens of MHz. Adopting a high frequency $f_{us}$ allows spatial resolution to increase. However, in the case of the living body 17, if the frequency is increased, attenuation in the living body 17 also increases.

Fig. 1 schematically shows an arrangement state in which the ultrasound transducer 2 is separated apart from the surface of the living body 17, but in the case of such a separated arrangement, an ultrasound transmission member or ultrasound transmission medium that transmits ultrasound (with little loss) is inserted between the ultrasound transducer 2 (not shown) and the living body 17. Ultrasound may also be emitted from the surface of the living body 17 into the interior thereof without inserting any ultrasound transmission member or the like.

Furthermore, the pulse generator 13 outputs a timing pulse shown in Fig. 3 which is a delayed high frequency pulse to the signal processing apparatus 8. The delay time is on the order of time in which the ultrasound reaches the region to be examined 18 after pulsed ultrasound is emitted from the ultrasound transducer 2. This time is represented by Td in Fig. 3.

In other words, for the signal processing apparatus 8, timing at which a pulse delayed by this delay time is inputted corresponds to timing at which light modulated through ultrasound vibration by the region to be examined 18 (which is moved through scanning) is detected as a detected signal of scattered light by the light detector 7. That is, this pulse has a function of timing pulse for detected signal to be detected.

The signal processing apparatus 8 then incorporates the detected signal from timing of, for example, a rising edge of the timing pulse, further performs signal processing such as Fourier transform (represented as signal processing in Fig. 3), calculates a degree of modulation m and outputs the degree of modulation m to the PC 9.

Furthermore, upon ending signal processing (or incorporation of the detected signal) of calculating the degree of modulation m, the signal processing apparatus 8 outputs a scanning control signal (signal for moving to the next region to be examined 18) shown in Fig. 3 to the scanning driver 14.

Upon receiving the scanning control signal, the scanning driver 14 outputs a drive signal for moving the scanning section 3 by one step. The scanning section 3 can perform scanning so as to cover, for example, an XY plane, that is, a two-dimensional scanning range along the horizontal surface. Scanning can be realized on not only the XY plane but also the XZ plane or YZ plane.

Therefore, the signal processing apparatus 8 receives a detected signal of scattered light as input with the position of the region to be examined (also referred to as a "scanning position") moved (scanned) little by little as a local region subjected to ultrasound irradiation and light irradiation.

The above described PC 9 stores the volume fraction $X_p$ and N/C ratio that are feature information on the constituent tissues at the scanning position of the region to be examined 18 in the memory 12 in association with the scanning position of the region to be examined 18 from the degree of modulation m inputted from the signal processing apparatus 8 and also outputs the volume fraction $X_p$ and N/C ratio as images to the monitor 16 as display means.

The present embodiment describes a case where both the volume fraction $X_p$ and the N/C ratio are calculated as feature information, but only one of the two may be calculated. Furthermore, in the case where the volume fraction $X_p$ and the N/C ratio are displayed as images, the present invention is not limited to the case where both images are displayed, but only one of the two may be displayed. Furthermore, one or both selected by the operator et al. may be displayed as an image.

Furthermore, the memory 12 of the PC 9 stores, in a table form, the nature or state of cell tissue, that is, information on thresholds $X_{th}$ and $(N/C)_{th}$ for discriminating both tissues from the precalculated volume fraction $X_p$ or N/C ratio in the case of normal tissue and lesioned tissue in an organ or region having different (cell) tissue properties. $X_{th}$ denotes a threshold in the case of volume fraction $X_p$ and $(N/C)_{th}$ denotes a threshold in the case of N/C ratio.

A discriminating section 9c as discriminating means of the PC 9 discriminates actually measured properties of the constituent tissues of the region to be examined 18 using the threshold $X_{th}$ or $(N/C)_{th}$ of feature information for discriminating both tissues stored in the memory 12.

In this case, the discriminating section 9c compares the volume fraction $X_p$ or N/C ratio as feature information calculated based on the degree of modulation m or the like calculated through detection of scattered light from the region to be examined 18 with the threshold $X_{th}$ or $(N/C)_{th}$ as reference information calculated beforehand to perform discrimination using a comparator or the like and discriminates tissue properties as to whether the tissue is a normal tissue or a lesioned tissue from the comparison result.

To be more specific, the discriminating section 9c of the PC 9 discriminates the tissue as a normal tissue when

the volume fraction $X_p$ is less than the threshold $X_{th}$ and discriminates the tissue as a lesioned tissue when the volume fraction $X_p$ is equal to or above the threshold $X_{th}$.

Likewise, the discriminating section 9c discriminates the tissue as a normal tissue when the N/C ratio is less than the threshold $(N/C)_{th}$ and discriminates the tissue as a lesioned tissue when the N/C ratio is equal to or above threshold $(N/C)_{th}$.

Furthermore, the discriminating section 9c of the PC 9 stores the information of the discrimination result (also referred to as "discrimination information") added to or in association with the volume fraction $X_p$ or N/C ratio as the feature information in the memory 12.

When the PC 9 displays the feature information stored in the memory 12 on the monitor 16 as an image, the PC 9 performs processing of adding and displaying the information of the discrimination result as well. That is, an image display processing section 9d of the PC 9 has a function of display processing means for displaying feature information as an image with the discrimination information of the discrimination result on the feature information added as well.

In this case, when the feature information is less than the threshold, the image display processing section 9d of the PC 9 displays the feature information with, for example, a monochrome brightness value and displays the feature information colored in red when the feature information is equal to or above the threshold. In the case of coloring, the color is not limited to red.

That is, the PC 9 performs image display processing of displaying the feature information of each region to be examined 18 (scanning position) in the scanning range on the monitor 16 with information on the discrimination result added.

The operator can know that the portion displayed, for example, in red is more likely to be a lesioned tissue from information displayed as an image on the monitor 16, diagnose the portion carefully and thereby more easily make an efficient diagnosis.

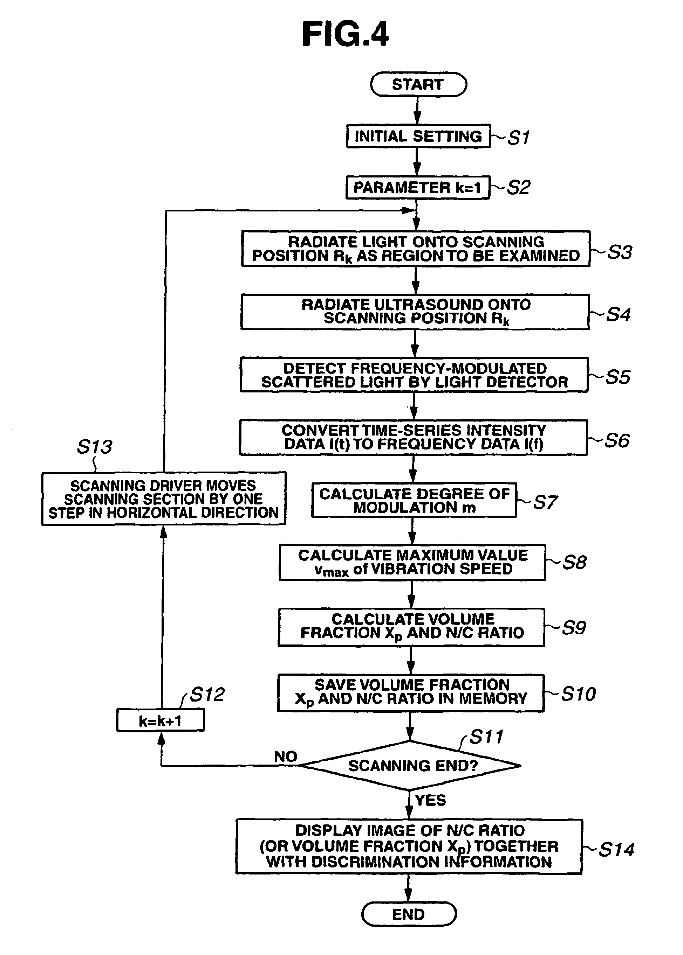

Next, the operation of the biological information analyzing apparatus 1 of the present embodiment, in other words, the processing procedure of the biological information analyzing method as the first embodiment of the subject information analyzing method will be described with reference to Fig. 4. In first step S1, the operator makes an initial setting.

In this initial setting, the operator specifies an organ (or region) in the living body 17 to be diagnosed or examined or inputs information necessary to calculate the degree of modulation m, maximum value $v_{max}$ of vibration speed and volume fraction $X_p$ or the like. The inputted information is stored, for example, in the memory 12, the information is stored and referenced as required.

In this case, the operator may also be enabled to select corresponding information from a list of information provided beforehand in the biological information analyzing apparatus 1 to make the initial setting.

In next step S2, for example, the signal processing apparatus 8 sets parameter k indicating the scanning position of the region to be examined 18 to initial value k=1. Here, parameter k includes, for example, a parameter of the scanning range in the X direction and a parameter of the scanning range in the Y direction, which are to be a scanning range on the horizontal plane.

In next step S3, light from the light source apparatus 4 is radiated at a scanning position $R_k(=R_1)$ of the initial setting in the region to be examined 18 of the living body 17 after passing through the optical fiber 5. The scanning position $R_k$ represents the three-dimensional position coordinates at $R_k(x, y, z)$ in an abbreviated manner.

As shown in step S3, the light source apparatus 4 can use a laser diode that generates, for example, laser light. The laser light may be continuous light or pulsed light. Fig. 3 illustrates a case of continuous light.

Furthermore, as shown in step S4, the ultrasound transducer 2 emits pulsed ultrasound based on a high frequency pulse generated, for example, in a predetermined cycle from the pulse generator 13. The emitted ultrasound is focused at the scanning position $R_k$, that is, at each region to be examined 18.

At the scanning position $R_k$, the living body constituent tissues are vibrated by ultrasound and laser light is scattered by the ultrasound vibrated living body constituent tissues.

As shown in step S5, light intensity I of scattered light frequency-modulated through ultrasound vibration passes through the optical fiber 6 and is detected (received) by the light detector 7. The light detector 7 outputs the detected signal to the signal processing apparatus 8.

In other words, in the processing in step S5, the light detector 7 as light receiving means receives the frequency-modulated light as scattered light corresponding to the irradiating light of a second frequency from the region to be examined 18 of the living body 17 irradiated with ultrasound of the first frequency.

The signal processing apparatus 8 incorporates this detected signal in synchronization with the timing pulse from the pulse generator 13, A/D-converts the signal and stores the signal in a memory in the signal processing apparatus 8.

Furthermore, as shown in step S6, the Fourier transform circuit 8a in the signal processing apparatus 8 transforms the detected signal of light intensity I of the scattered light from A/D converted time-series intensity data I(t) to frequency data I(f). Here, f indicates that the signal data I(f) is signal data using the frequency as a variable.

Furthermore, I and I(t) are the same except for the difference as to whether they are an analog amount or a digital amount. The Fourier transform circuit 8a separates and extracts spectral intensity as intensity information of a specific frequency $Lf_{us}$ shifted by an integer multiple L of the frequency $f_{us}$ of ultrasound as shown on the right side of equation (3) from intensity data I(t). Therefore, the processing in step S6 has a function of the information extracting step of extracting information on a specific frequency shifted by an integer multiple L of the frequency $f_{us}$ of ultrasound.

As shown in step S7, the degree of modulation calculation section 8b of the signal processing apparatus 8 calculates the degree of modulation m as information on a specific frequency from the intensity information on a specific frequency according to, for example, equation (13). This is, however, not limited to the case where the degree of modulation m is calculated from equation (13). The signal processing apparatus 8 outputs the calculated information of the degree of modulation m to the PC 9.

As shown in step S8, the PC 9 calculates the maximum value $v_{max}$ of the vibration speed of nuclei corresponding to particles in equation (4) using information on the degree of modulation m, angle $\alpha$, wavelength $\lambda_o$ of light, frequency $f_{us}$ of ultrasound or the like stored in the memory 12.

Furthermore, in next step S9, the PC performs fitting on the calculated maximum value $v_{max}$ of the vibration speed using the volume fraction $X_p$ occupied by nuclei as a parameter taking advantage of the relationship in equation (5) and using equation (6) and equation (7).

The PC 9 then calculates the volume fraction $X_p$ of nuclei as the feature information corresponding to the calculated maximum value $v_{max}$ of the vibration speed from the value of the parameter that fits most in step S9.

It may also be possible to store in a table form and provide beforehand the value of volume fraction $X_p$ that fits when changing the values of maximum value $v_{max}$ of the vibration speed, ultrasound intensity $I_{us}$, velocity of sound $V_{us}$ or the like so as to satisfy the relationships in (5), (6) and (7). In this case, the value of volume fraction $X_p$ can be calculated with reference to the table.

Furthermore, after calculating the volume fraction $X_p$, the PC 9 calculates the N/C ratio as feature information of the composition ratio using this volume fraction $X_p$. That is, in step S9, the PC 9 calculates the volume fraction and N/C ratio as feature information of the constituent tissues of the region to be examined 18 based on the information on a specific frequency.

Furthermore, as shown in step S10, the PC 9 saves (stores), in the memory 12, the calculated volume fraction $X_p$ and N/C ratio in association with the scanning position $R_k$ (or parameter k). In this case, the PC 9 also saves information on the discrimination result by the discriminating section 9c in the memory 12.

In next step S 11, the signal processing apparatus 8 discriminates whether or not scanning has ended. When scanning has not ended, the signal processing apparatus 8 changes parameter k to k+1 as shown in step S12.

With the change of parameter k, the signal processing apparatus 8 outputs a scanning control signal shown in Fig. 3 for causing the scanning driver 14 to move by one step, for example, in the horizontal direction (X direction).

As shown in step S 13, the scanning driver 14 applies the drive signal shown in Fig. 3 to the piezoelectric element of the scanning section 3 and moves the scanning section by one step, for example, in the horizontal direction (X direction).

After this processing, the process returns to the processing in step S3. In step S3, the scanning position $R_k$ becomes $R_2$ and similar processing is performed on the scanning position $R_k$ (=$R_2$). Thus, when, for example, scanning over the predetermined scanning range in the X direction ends, similar scanning is performed in the Y direction as well.

When scanning in the X direction and the Y direction, that is, scanning in a two-dimensional predetermined scanning range ends, the process moves to processing in step S 14 next to step S11.

In this step S 14, the PC 9 reads the information on the volume fraction $X_p$ and N/C ratio value as feature information in the predetermined scanning range saved in the memory 12 and discrimination information thereof. The PC 9 then displays, for example, a brightness value corresponding to the volume fraction $X_p$ or N/C ratio value and the discrimination information thereof as feature information in the scanning range on the display screen of the monitor 16 as images.

That is, in step S 14, the PC 9 images and displays the N/C ratio as feature information for discriminating tissue properties of the living body constituent tissues of the region to be examined 18 based on the calculated volume fraction $X_p$, and then ends the processing shown in Fig. 4.

When the N/C ratio is imaged and displayed, for example, the brightness value of density $\rho$ at the position is displayed in monochrome, but when the density $\rho$ is equal to or above a threshold $\rho_{th}$, the density at the position is displayed in red.

Fig. 1 illustrates an overview of display information 16a which is the brightness value of, for example, N/C ratio in the scanning range imaged with its discrimination information added thereto on the display screen of the monitor 16. An N/C ratio equal to or above the threshold $(N/C)_{th}$ is calculated in the vicinity of the center in the scanning range, and therefore the N/C ratio is displayed in red (black circles in the figure) and the N/C ratio less than $(N/C)_{th}$ is calculated in the rest of the area, which is displayed in monochrome (white circles in the figure).

The operator can know that the portion displayed in red is likely to be a lesioned tissue from the display information 16a displayed as an image on the monitor 16, and can make an efficient diagnosis by diagnosing the portion carefully.

Thus, according to the present embodiment, when detecting scattered light at the time of ultrasound irradiation, it is possible to generate a volume fraction and a nuclei/cytoplasm ratio (N/C ratio) as feature information on the composition ratio of constituent tissues of the region to be examined without requiring high frequency resolution on frequency-modulated light and discriminate properties of the constituent tissues of the region to be examined.

Furthermore, the present embodiment does not require high frequency resolution, and can thereby generate a volume fraction and nuclei cytoplasm ratio (N/C ratio) as feature information on the composition ratio of constituent tissues of the region to be examined or discriminate properties of constituent tissues of the region to be examined without taking much time for signal processing or without requiring any expensive apparatus.

Furthermore, the present embodiment displays the imaged volume fraction and N/C ratio and further displays them with information on the discrimination result added, and can thereby provide the operator et al. with an environment in which it is easier to make an efficient diagnosis.

(Second embodiment)

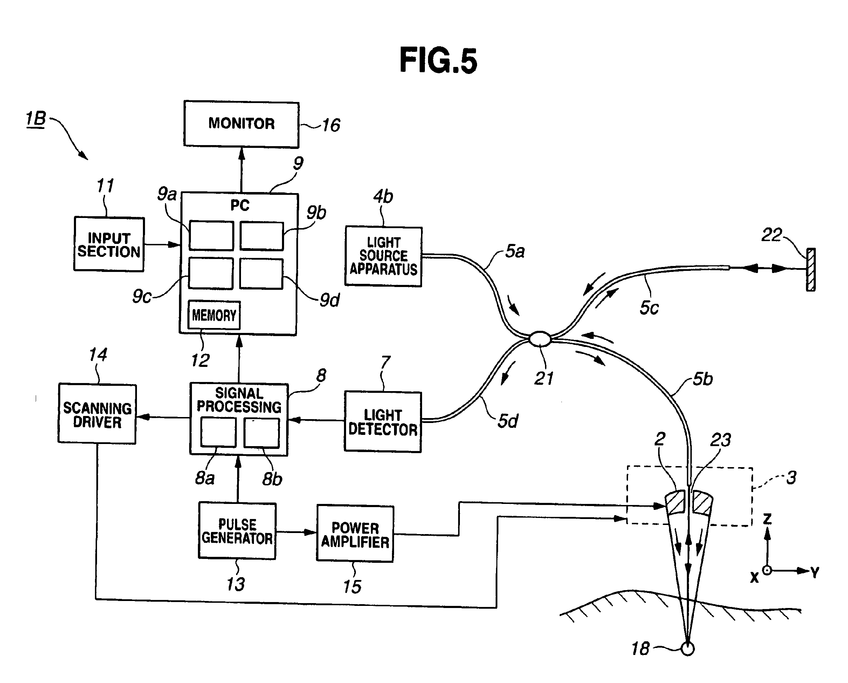

Next, a biological information analyzing apparatus 1B according to a second embodiment of a subject information analyzing apparatus of the present invention will be described with reference to Fig. 5. Fig. 5 illustrates a configuration of the biological information analyzing apparatus 1B of the second embodiment. The biological information analyzing apparatus 1B basically adopts a configuration with means for improving a signal to noise ratio added to the biological information analyzing apparatus 1 of the first embodiment.

Light of a light source apparatus 4b in the present embodiment is impinged at one end of an optical fiber 5a, an optical coupler 21 is provided at some midpoint of the optical fiber 5a and the light guided by the optical fiber 5a is branched at the optical coupler 21 to an optical fiber 5b extending on a scanning section 3 side and an optical fiber 5c extending on a reference mirror 22 side.

A distal end portion of the optical fiber 5b is mounted in the scanning section 3 as in the first embodiment. However, in the embodiment in Fig. 5, the distal end portion of the optical fiber 5b is arranged so as to face an opening 23 provided, for example, in the center of an ultrasound transducer 2.

The optical fiber 5b emits the light from the light source apparatus 4b from the distal end portion thereof to a region to be examined 18 and scattered light scattered by the region to be examined 18 is impinged at a distal end portion of the optical fiber 5b.

Therefore, the optical fiber 5b has a dual function as the optical fiber 5 and the optical fiber 6 in the first embodiment. Part of the scattered light impinged on the optical fiber 5b is impinged on an optical fiber 5d that guides light to a light detector 7 from the optical coupler 21.

Furthermore, light guided from the light source apparatus 4b, through the optical coupler 21 to the optical fiber 5c is reflected by the reference mirror 22 oppositely arranged at a distal end of the optical fiber 5c and returns to the optical coupler 21 again.

In this case, such a setting is made that an optical path length of light (optical path length for reference light) guided from the optical coupler 21 to the optical fiber 5c side, reflected by the reference mirror 22 and returning to the optical coupler 21 as reference light substantially matches an optical path length of light (optical path length for measuring light) guided from the optical coupler 21 to the optical fiber 5b side, scattered by the region to be examined 18, passing through the optical fiber 5b and returning to the optical coupler 21 (as measuring light for a measuring target).

Furthermore, according to the present embodiment, the light generated by the light source apparatus 4b is, for example, low interference light and this low interference light is light with a short coherent length that no longer interferes when the above described difference in optical path length is equal to or more than on the order of several tens of $\mu$m.

Therefore, of the light emitted from the light source apparatus 4b, passing through the optical fiber 5a, branched to the optical fiber 5b and the optical fiber 5c, passing through the optical fiber 5b, scattered on the region to be examined 18 side and impinged on the optical fiber 5b again, only the scattered light from the vicinity of the region to be examined 18 corresponding to the case with an optical path length substantially equal to the optical path length for reference light interferes with the reference light.

As described in the first embodiment, scattered light has a spectrum shifted by an integer multiple of angular frequency $\omega_{us}$ of ultrasound as expressed by equation (3) due to a Doppler effect from an angular frequency $\omega_o$ of light (before scattering). Interference light optically interfering with reference light becomes heterodyne detected light of low frequency of $\pm 1*\omega_{us}$, $\pm 2*\omega_{us}$, $\pm 3*\omega_{us}$, ... which corresponds to a light component of a difference in common angular frequency $\omega_o$ in both reference light and interference light.

This interference light is received by the light detector 7 through the optical fiber 5d, photoelectrically converted to a detected signal and inputted to the signal processing apparatus 8. In the first embodiment, information on a specific frequency is electrically extracted from a detected signal received by the light detector 7. By contrast, the present embodiment adopts a configuration in which information on a specific frequency is optically extracted by causing scattered light to interfere with reference light. That is, the light detector as light receiving means of the present embodiment has a function as interference light receiving means for receiving interference light between scattered light and reference light. In a modification example which will be described later, interference light receiving means having a similar function in a simpler configuration is formed.

Furthermore, in the present embodiment, the ultrasound transducer 2 radiates ultrasound onto the region to be examined 18 and the angle $\alpha$ formed between the advancing direction of ultrasound that causes the living body constituent tissues in the region to be examined 18 to ultrasound vibrate and the light irradiating and detecting direction is substantially 0, that is, both directions substantially match.

The signal processing apparatus 8 separates (transforms), for example, a detected signal subjected to heterodyne detection as in the case of the first embodiment to frequency data through Fourier transform and calculates a degree of modulation m from the intensity of a specific frequency in that case.

The rest of the configuration is similar to that of the first embodiment and descriptions thereof will be omitted.

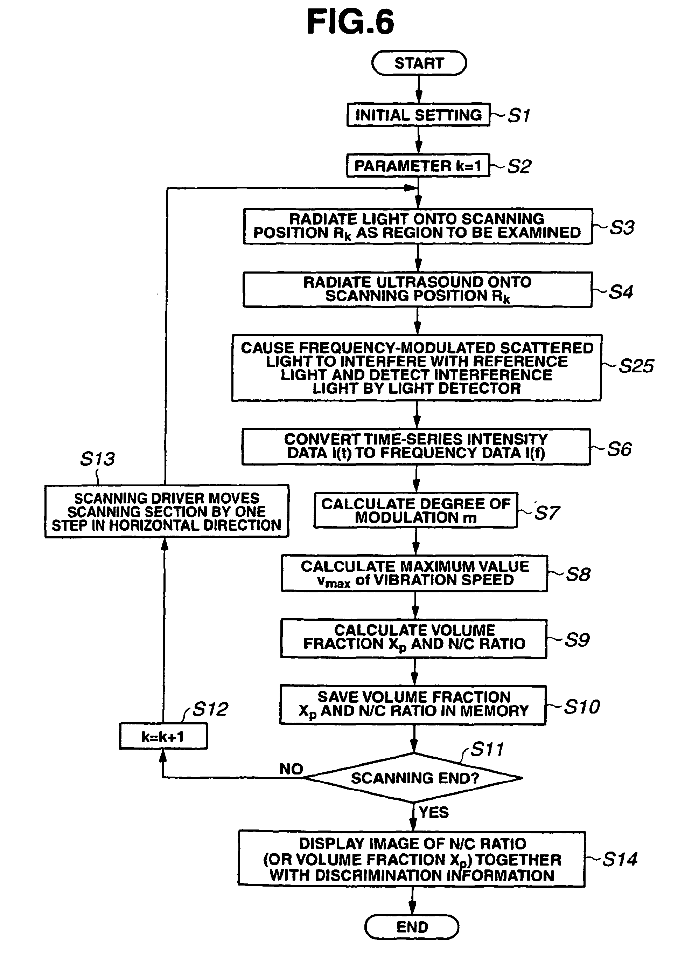

Furthermore, the present embodiment performs operation similar to that of the first embodiment by reading (substituting) "detecting scattered light" in the first embodiment as "detecting heterodyne detected interference light." Fig. 6 illustrates operation of the present embodiment, in other words, a flowchart of a typical example of processing procedure of the biological information analyzing method according to the second embodiment of the subject information analyzing method.

In the flowchart shown in Fig. 6, the processing in step S5 in the flowchart of Fig. 4 is changed to processing in step S25. In step S25, frequency-modulated scattered light interferes with reference light reflected by the reference mirror 22, and is detected by the light detector 7 as interference light which is a frequency component by far lower than frequency $f_0$ of light. The light detector 7 then outputs the detected signal to the signal processing apparatus 8.

The signal processing apparatus applies Fourier transform to a detected signal in a sufficiently lower signal band than that in the first embodiment as shown in step S6 as in the case of the first embodiment. In next step S7, the degree of modulation m is calculated. Using the calculated degree of modulation m, processing such as step S8 similar to the processing shown in Fig. 4 is performed. The monitor 16 displays an image ofN/C ratio with discrimination information added thereto.

The present embodiment causes scattered light to interfere with reference light of a frequency $f_0$ of light so as to detect an interference light component in a sufficiently low signal band, and can thereby calculate a degree of modulation m as information on a specific frequency more accurately. The volume fraction $X_p$ and N/C ratio as feature information can be calculated from the degree of modulation m.

Furthermore, since the signal band of interference light is a sufficiently lower band than that of the first embodiment, it is possible to use a signal processing apparatus slower than that in the first embodiment. In other aspects, the second embodiment has effects similar to those of the first embodiment.

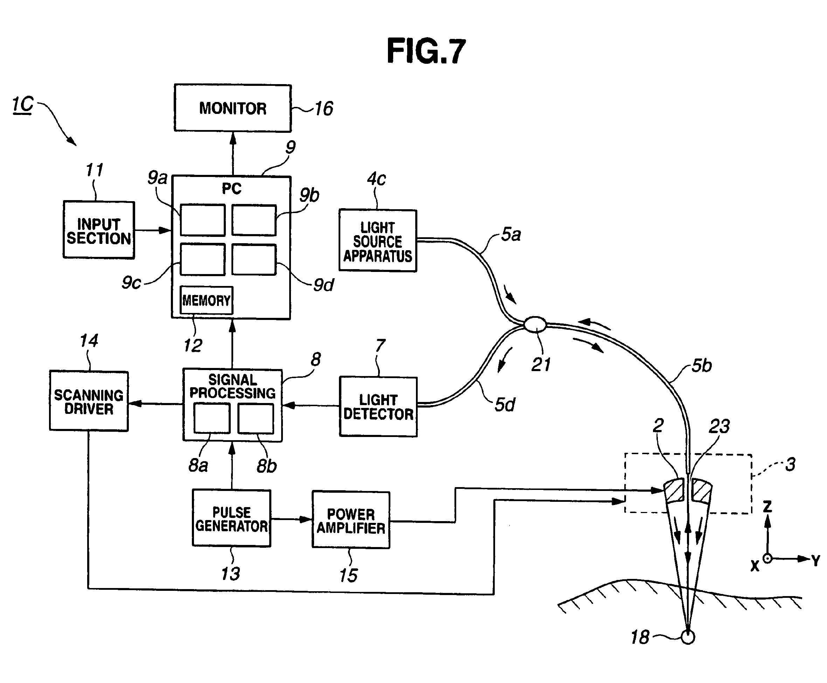

Next, a biological information analyzing apparatus 1C according to a modification example of the present embodiment will be described with reference to Fig. 7. The biological information analyzing apparatus 1C adopts a configuration without the optical fiber 5c and the reference mirror 22 for generating reference light in the biological information analyzing apparatus 1B shown in Fig. 5. Furthermore, the present modification example adopts a light source apparatus 4c instead of the light source apparatus 4b in the living body observation apparatus 1B shown in Fig. 5. The light source apparatus 4c generates, for example, laser light by a laser diode (LD) or LED light by a light-emitting diode (LED).

The second embodiment in Fig. 5 adopts the configuration that causes scattered light to interfere with reference light reflected by the reference mirror 22 different from the scattered light side. In this case, the optical path length for reference light is set to the value of the optical path length for measuring light in order to selectively interfere with only the scattered light from the vicinity of the region to be examined 18 and a low interference light source having a sufficiently short coherent length is used.

By contrast, the present modification example uses a light source such as LD that generates light having a longer coherent length than that of low interference light of a low interference light source and detects interference light between scattered light from the vicinity of the region to be examined 18 and scattered light of peripheral part of the region to be examined 18.

That is, since the peripheral part of the region to be examined 18 is out of the focus position of focused ultrasound, the scattered light scattered in the peripheral part becomes scattered light not accompanied by frequency shifting from the frequency f of the incident light. That is, since the peripheral part is substantially not vibrating ultrasonically, the scattered light in the peripheral part is not accompanied by frequency shifting.

The interference light resulting from interference between the frequency-modulated scattered light from the region to be examined 18 on which ultrasound is focused and the scattered light not accompanied by frequency shifting is also impinged on the optical fiber 5b and the light detector 7 detects the interference light component as a detected signal. Signal processing on the detected signal detected by the light detector 7 is similar to the case with Fig. 5.

According to the present modification example, it is possible to obtain substantially the same operation and effect in a simpler configuration than the case with Fig. 5.

The present invention may also adopt a configuration obtained by modifying the aforementioned embodiments. For example, such a configuration may be adopted that the signal processing apparatus 8 in the first embodiment includes the function of the PC 9.

Furthermore, in this case, such a configuration may be adopted that a control program storage section that stores a control program for operating the biological information analyzing apparatus 1 according to the processing procedure of the flowchart shown in Fig. 4 is provided in the signal processing apparatus 8. A configuration may also be adopted in which the PC 9 includes the function of the signal processing apparatus 8.

Furthermore, a configuration may also be adopted in which the scanning section 3 can perform three-dimensional scanning. Furthermore, a configuration may also be adopted in which an arbitrary three-dimensional plane is selectively set so as to enable an analysis or observation including discrimination of tissue properties of the living body 17 to be realized. In the case where the configuration in Fig. 5 is used, when scanning is performed in the depth direction, the reference mirror 22 may also be moved in conjunction with scanning in the depth direction.

By contrast, when the configuration in Fig. 7 is used instead of the configuration in Fig. 5, the reference mirror 22 need no longer be moved and there is also an advantage that the configuration of the control system can also be simplified. An embodiment configured by partially combining the aforementioned embodiments or the like also belongs to the present invention.

**Claims**

1. A subject information analyzing apparatus (1, 1B, 1C) comprising:

   ultrasound irradiation means (2) that irradiates a subject (17) with ultrasound of a first frequency;
   light irradiating means (4, 5; 4b, 5a, 5b; 4c, 5a, 5b) that irradiates a region to be examined (18) in the subject (17) with light of a second frequency;
   light receiving means (6, 7; 5d, 7) that receives frequency-modulated light as scattered light corresponding to irradiating light of the second frequency from the region to be examined (18) in accordance with a Doppler effect through vibration of constituent tissues making up the region to be examined (18) provoked by the ultrasound of the first frequency; and
   information extracting means (8a, 8b, 9a) that extracts intensity information on a specific frequency shifted by an integer multiple of the first frequency from the second frequency from the frequency-modulated light received by the light receiving means (6, 7; 5d, 7), **characterized in that** the subject information analyzing apparatus (1, 1B, 1C) further comprises
   feature information generating means that generates, as feature information on a composition ratio of the constituent tissues making up the region to be examined (18) when the constituent tissues are made up of cell nuclei and cytoplasm, a calculated value of a volume fraction indicating a volume proportion occupied by the cell nuclei or a cell nuclei/cytoplasm ratio using a degree of modulation calculated by the information extracting means (8a, 8b, 9a), Wherein
   the information extracting means (8a, 8b, 9a) calculates the degree of modulation of the frequency-modulated light used to generate the feature information using intensity information with at least two different specific frequencies shifted by an integer multiple of the first frequency from the second frequency.

2. The subject information analyzing apparatus (1, 18, 1C) according to claim 1, further comprising:

   scanning means (3) that scans the region to be examined (18) irradiated with then ultrasound of the first frequency and also irradiated with the illuminating light of the second frequency; and
   display processing means (9d) that displays calculated values of the volume fraction or the cell nuclei/cytoplasm ratio of constituent tissues making up the region to be examined (18) of each scanned position as an image.

3. The subject information analyzing apparatus (1, 1B, 1C) according to claim 1, wherein the light receiving means (6, 7; 5d, 7) is made up of interference light receiving means (5c, 22; 5d, 7) that receives interference light resulting from causing frequency-modulated light as the scattered light from the region to be examined (18) irradiated with the focused ultrasound to interfere with reference light resulting from causing the irradiating light to be reflected and not accompanied by frequency modulation.

4. The subject information analyzing apparatus (1, 1B, 1C) according to claim 1, wherein the light receiving means (6, 7; 5d, 7) is made up of interference light receiving means (5d, 7) that receives interference light resulting from causing frequency-modulated light as the scattered light from the region to be examined (18) irradiated with the focused ultrasound to interfere with scattered light substantially not accompanied by frequency modulation from an area irradiated with substantially no ultrasound in a periphery of the region to be examined (18).

5. The subject information analyzing apparatus (1, 1B, 1C) according to claim 1, wherein the information extracting means (8a, 8b, 9a) calculates a maximum value of vibration speed of the cell nuclei making up the constituent tissues irradiated with the ultrasound based on the information on a degree of modulation.

6. A subject information analyzing apparatus (1, 1B, 1C) according to claim 1, further comprising:

discriminating means (9) that discriminates properties of constituent tissues based on feature information on a composition ratio of the constituent tissues making up the region to be examined (18) calculated using the information on the specific frequency extracted by the information extracting means (8a, 8b, 9a).

7. The subject information analyzing apparatus (1, 1B, 1C) according to claim 6, wherein the discriminating means (9c) discriminates properties of the constituent tissues by comparing the calculated value of the feature information with a threshold calculated beforehand.

8. The subject information analyzing apparatus (1, 1B, 1C) according to claim 6, further comprising:

scanning means (3) that scans the region to be examined (18) irradiated with illuminating light of a predetermined frequency by the light irradiating means (4, 5; 4b, 5a, 5b; 4c, 5a, 5b) and also irradiated with the ultrasound of the first frequency by the ultrasound irradiation means (2); and
display processing means (9d) that displays, as an image, information of a discrimination result of discriminating tissue properties in constituent tissues making up the region to be examined (18) of each scanned position.

9. A subject information analyzing method comprising:

a light irradiation step of light irradiating means (4, 5; 4b, 5a, 5b; 4c, 5a, 5b) irradiating a region to be examined (18) in a subject (17) with light of a second frequency;
an ultrasound irradiating step of ultrasound irradiating means (2) irradiating the subject (17) with ultrasound of a first frequency;
a light receiving step of light receiving means (6, 7; 5d, 7) receiving frequency-modulated light as scattered light corresponding to irradiating light of the second frequency from the region to be examined (18) in accordance with a Doppler effect through vibration of constituent tissues of constituent tissues making up the region to be examined (18) provoked by the ultrasound of a first frequency; and
an information extracting step of information extracting means (8a, 8b, 9a) extracting intensity information on a specific frequency shifted by an integer multiple of the first frequency from the second frequency from the frequency-modulated light received by the light receiving step, **characterized in that** the method further comprises:
a volume fraction calculating step of volume fraction calculating means (9b) calculating, as feature information on a composition ratio of the constituent tissues making up the region to be examined when the constituent tissues are made up of cell nuclei and cytoplasm, a calculation value of a volume fraction indicating a volume proportion occupied by the cell nuclei based on a degree of modulation calculated in the information extracting step; and
a displaying step of display means (16) displaying a cell nuclei/cytoplasm ratio calculated on the basis of the volume fraction calculated in the volume fraction calculating step, wherein
the information extracting step calculates the degree of modulation of the frequency-modulated light for calculating the calculation value of a volume fraction from intensity information with at least two different specific frequencies shifted by an integer multiple of the first frequency from the second frequency.

**Patentansprüche**

1. Vorrichtung (1, 1B, 1C) zur Analyse von Patienteninformationen, die aufweist:

eine Ultraschallbestrahlungseinrichtung (2), die einen Patienten (17) mit Ultraschall einer ersten Frequenz bestrahlt;

eine Lichtbestrahlungseinrichtung (4, 5; 4b, 5a, 5b; 4c, 5a, 5b), die ein zu untersuchendes (18) Gebiet im Patienten (17) mit Licht einer zweiten Frequenz bestrahlt;

eine Lichtempfangseinrichtung (6, 7; 5d, 7), die frequenzmoduliertes Licht als Streulicht empfängt, das einem Bestrahlungslicht der zweiten Frequenz von dem zu untersuchenden Gebiet (18) gemäß einem Dopplereffekt durch Vibration der das zu untersuchende Gebiet (18) bildenden Gewebebestandteile, die durch den Ultraschall der ersten Frequenz ausgelöst wurde, entspricht; und

eine Informationsextrahierungseinrichtung (8a, 9b, 9a), die Intensitätsinformationen zu einer speziellen Frequenz, die um ein ganzzahliges Vielfaches der ersten Frequenz von der zweiten Frequenz von dem von der Lichtempfangseinrichtung (6, 7; 5b, 7) empfangenen frequenzmodulierten Licht verschoben ist, extrahiert,

**dadurch gekennzeichnet, dass** die Vorrichtung zur Analyse von Patienteninformationen (1, 1B, 1C) weiterhin aufweist

eine Einrichtung zum Generieren von Merkmalsinformation, die als Merkmalsinformation zu einem Zusammensetzungsverhältnis der das zu untersuchende Gebiet (18) bildenden Gewebebestandteile, wenn die Gewebebestandteile aus Zellkernen und Zytoplasma bestehen, einen berechneten Wert eines Volumenanteils, der ein Volumenverhältnis angibt, das von den Zellkernen oder einem Zellkern/Zytoplasma-Verhältnis belegt wird, unter Verwendung eines von der Informationsextrahierungseinrichtung (8a, 8b, 9a) berechneten Modulationsgrades generiert,

wobei die Informationsextrahierungseinrichtung (8a, 8b, 9a) den Modulationsgrad des frequenzmodulierten Lichts berechnet, mit dem die Merkmalsinformation unter Verwendung der Intensitätsinformationen mit wenigstens zwei unterschiedlichen speziellen Frequenzen, die um ein ganzzahliges Vielfaches der ersten Frequenz von der zweiten Frequenz verschoben sind, generiert wird.

2. Vorrichtung (1, 1B, 1C) zur Analyse von Patienteninformationen nach Anspruch 1, die weiterhin aufweist:

eine Abtasteinrichtung (3), die das zu untersuchende Gebiet (18) abtastet, das mit dem Ultraschall der ersten Frequenz bestrahlt wird und auch mit dem Beleuchtungslicht der zweiten Frequenz bestrahlt wird; und

eine Anzeigeverarbeitungseinrichtung (9d), die berechnete Werte des Volumenanteils oder des Zellkern/Zytoplasma-Verhältnisses der Gewebebestandteile, die den zu untersuchenden Bereich (18) bilden, für jede abgetastete Position als Bild anzeigt.

3. Vorrichtung (1, 1B, 1C) zur Analyse von Patienteninformationen nach Anspruch 1, wobei die Lichtempfangseinrichtung (6, 7; 5d, 7) aus einer Interferenzlichtempfangseinrichtung (5c, 22; 5d, 7) besteht, die Interferenzlicht empfängt, das dadurch entsteht, dass frequenzmoduliertes Licht als das Streulicht aus dem zu untersuchenden Gebiet (18), das mit dem fokussierten Ultraschall bestrahlt wird, mit Referenzlicht, das entsteht, indem bewirkt wird, dass das Bestrahlungslicht reflektiert wird und nicht von der Frequenzmodulation betroffen ist, zur Interferenz gebracht wird.

4. Vorrichtung (1, 1B, 1C) zur Analyse von Patienteninformationen nach Anspruch 1, wobei die Lichtempfangseinrichtung (6, 7; 5d, 7) aus einer Interferenzlichtempfangseinrichtung (5d, 7) besteht, die Interferenzlicht empfängt, das dadurch entsteht, dass frequenzmoduliertes Licht als das Streulicht aus dem zu untersuchenden Gebiet (18), das mit dem fokussierten Ultraschall bestrahlt wird, mit Streulicht, das im Wesentlichen nicht von der Frequenzmodulation betroffen ist, aus einem im Wesentlichen nicht mit Ultraschall bestrahlten Bereich in einem Randbereich des zu untersuchenden Gebiets (18) zur Interferenz gebracht wird.

5. Vorrichtung (1, 1B, 1C) zur Analyse von Patienteninformationen nach Anspruch 1, wobei die Informationsextrahierungseinrichtung (8a, 8b, 9a) einen Maximalwert der Vibrationsgeschwindigkeit der Zellkerne, die die mit dem Ultraschall bestrahlten Gewebebestandteile bilden, basierend auf der Information eines Modulationsgrades berechnet.

6. Vorrichtung (1, 1B, 1C) zur Analyse von Patienteninformationen nach Anspruch 1, die weiterhin aufweist:

eine Unterscheidungseinrichtung (9), die Eigenschaften der Gewebebestandteile unterscheidet, basierend auf Merkmalsinformation zu einem Zusammensetzungsverhältnis der das zu untersuchende Gebiet (18) bildenden Gewebebestandteile, das unter Verwendung der Information zu der von der Informationsextrahierungseinrichtung (8a, 8b, 9a) extrahierten speziellen Frequenz berechnet wird.

7. Vorrichtung (1, 1B, 1C) zur Analyse von Patienteninformationen nach Anspruch 6, wobei die Unterscheidungseinrichtung (9c) Eigenschaften der Gewebebestandteile unterscheidet, indem der berechnete Wert der Merkmalsinformation mit einem zuvor berechneten Grenzwert verglichen wird.

8.  Vorrichtung (1, 1B, 1C) zur Analyse von Patienteninformationen nach Anspruch 6, die weiterhin aufweist:

    eine Abtasteinrichtung (3), die das zu untersuchende Gebiet (18) abtastet, das mit Beleuchtungslicht einer bestimmten Frequenz durch die Lichtbestrahlungseinrichtung (4, 5; 4b, 5a, 5b; 4c, 5a, 5b) bestrahlt wird und auch mit dem Ultraschall der ersten Frequenz durch die Ultraschallbestrahlungseinrichtung (2) bestrahlt wird; und

    eine Anzeigeverarbeitungseichrichtung (9d), die Informationen eines Unterscheidungsergebnisses der Unterscheidung von Gewebeeigenschaften in den das zu untersuchende Gebiet (18) bildenden Gewebebestandteilen für jede abgetastete Position als ein Bild anzeigt.

9.  Verfahren zum Analysieren von Patienteninformationen, das aufweist:

    einen Lichtbestrahlungsschritt einer Lichtbestrahlungseinrichtung (4, 5; 4b, 5a, 5b; 4c, 5a, 5b), in dem ein zu untersuchendes (18) Gebiet in einem Patienten (17) mit Licht einer zweiten Frequenz bestrahlt wird;

    einen Ultraschallbestrahlungsschritt einer Ultraschallbestrahlungseinrichtung (2), in dem der Patient (17) mit Ultraschall einer ersten Frequenz bestrahlt wird;

    einen Lichtempfangsschritt einer Lichtempfangseinrichtung (6, 7; 5d, 7), in dem frequenzmoduliertes Licht als Streulicht empfangen wird, das einem Bestrahlungslicht der zweiten Frequenz aus dem zu untersuchenden Gebiet (18) gemäß dem Dopplereffekt durch die Vibration der das zu untersuchende Gebiet (18) bildenden Gewebebestandteile, die durch den Ultraschall der ersten Frequenz ausgelöst wurde, entspricht; und

    einen Informationsextrahierungsschritt einer Informationsextrahierungseinrichtung (8a, 9b, 9a), in dem Intensitätsinformationen zu einer speziellen Frequenz, die durch ein ganzzahliges Vielfaches der ersten Frequenz von der zweiten Frequenz von dem im Lichtempfangsschritt empfangenen, frequenzmodulierten Licht verschoben ist, extrahiert werden, **dadurch gekennzeichnet, dass** das Verfahren weiterhin aufweist:

    einen Schritt zum Berechnen des Volumenanteils einer Einrichtung (9b) zum Berechnen eines Volumenanteils, in dem als Merkmalsinformation zu einem Zusammensetzungsverhältnis der Gewebebestandteile, die das zu untersuchende Gebiet bilden, wenn die Gewebebestandteile aus Zellkernen und Zytoplasma bestehen, ein Berechnungswert eines Volumenanteils, der ein von den Zellkernen belegtes Volumenverhältnis angibt, basierend auf einem im Informationsextrahierungsschritt berechneten Modulationsgrad berechnet wird; und

    einen Anzeigeschritt einer Anzeigeeinrichtung (16), in dem ein Zellkern/Zytoplasma-Verhältnis, das auf der Basis des im Schritt zur Berechnung des Volumenanteils berechneten Volumenanteils berechnet wird, angezeigt wird, wobei

    der Informationsextrahierungsschritt den Modulationsgrad des frequenzmodulierten Lichts zum Berechnen des Berechnungswerts eines Volumenanteils aus Intensitätsinformationen mit wenigstens zwei unterschiedlichen speziellen Frequenzen berechnet, die um ein ganzzahliges Vielfaches der ersten Frequenz von der zweiten Frequenz verschoben sind.

## Revendications

1.  Appareil d'analyse d'information de sujet (1, 1B, 1C) comprenant :

    un moyen de projection d'ultrason (2) qui projette sur un sujet (17) un ultrason d'une première fréquence ;

    un moyen de projection de lumière (4, 5 ; 4b, 5a, 5b ; 4c, 5a, 5b) qui projette sur une région devant être examinée (18) dans le sujet (17) une lumière d'une deuxième fréquence ;

    un moyen de réception de lumière (6, 7 ; 5d, 7) qui reçoit une lumière modulée en fréquence comme une lumière diffusée correspondant à une lumière de projection de la deuxième fréquence à partir de la région devant être examinée (18) conformément à un effet Doppler par vibration de tissus constituants composant la région devant être examinée (18) provoqué par l'ultrason de la première fréquence ; et

    un moyen d'extraction d'information (8a, 8b, 9a) qui extrait une information d'intensité sur une fréquence spécifique décalée d'un multiple entier de la première fréquence par rapport à la deuxième fréquence de la lumière modulée en fréquence reçue par le moyen de réception de lumière (6, 7 ; 5d, 7), **caractérisé en ce que** l'appareil d'analyse d'information de sujet (1, 1B, 1C) comprend en outre

    un moyen de génération d'information de caractéristique qui génère, comme une information de caractéristique sur un rapport de composition des tissus constituants composant la région devant être examinée (18) lorsque les tissus constituants sont composés de noyaux cellulaires et de cytoplasme, une valeur calculée d'une fraction volumique indiquant une proportion volumique occupée par les noyaux cellulaires ou un rapport noyaux cellulaires/cytoplasme en utilisant un degré de modulation calculé par le moyen d'extraction d'information (8a, 8b, 9a), dans lequel

le moyen d'extraction d'information (8a, 8b, 9a) calcule le degré de modulation de la lumière modulée en fréquence utilisée pour générer l'information de caractéristique en utilisant une information d'intensité avec au moins deux fréquences spécifiques différentes décalées d'un multiple entier de la première fréquence par rapport à la deuxième fréquence.

2. Appareil d'analyse d'information de sujet (1, 1B, 1C) selon la revendication 1, comprenant en outre :

un moyen de balayage (3) qui balaye la région devant être examinée (18) sur laquelle est projeté l'ultrason de la première fréquence et est également projetée la lumière d'éclairage de la deuxième fréquence ; et
un moyen de traitement d'affichage (9d) qui affiche des valeurs calculées de la fraction volumique ou le rapport noyaux cellulaires/cytoplasme de tissus constituants composant la région devant être examinée (18) de chaque position balayée comme une image.

3. Appareil d'analyse d'information de sujet (1, 1B, 1C) selon la revendication 1, dans lequel le moyen de réception de lumière (6, 7 ; 5d, 7) est composé d'un moyen de réception de lumière d'interférence (5c, 22 ; 5d, 7) qui reçoit une lumière d'interférence résultant du fait qu'une lumière modulée en fréquence comme la lumière diffusée à partir de la région devant être examinée (18) sur laquelle l'ultrason focalisé est projeté interfère avec une lumière de référence résultant du fait que la lumière de projection est réfléchie et non accompagnée d'une modulation de fréquence.

4. Appareil d'analyse d'information de sujet (1, 1B, 1C) selon la revendication 1, dans lequel le moyen de réception de lumière (6, 7 ; 5d, 7) est composé d'un moyen de réception de lumière d'interférence (5d, 7) qui reçoit une lumière d'interférence résultant du fait qu'une lumière modulée en fréquence comme la lumière diffusée à partir de la région devant être examinée (18) sur laquelle l'ultrason focalisé est projeté interfère avec une lumière diffusée sensiblement non accompagnée d'une modulation de fréquence provenant d'une zone sur laquelle sensiblement pas d'ultrason n'est projeté dans une périphérie de la région devant être examinée (18).

5. Appareil d'analyse d'information de sujet (1, 1B, 1C) selon la revendication 1, dans lequel le moyen d'extraction d'information (8a, 8b, 9a) calcule une valeur maximum de vitesse de vibration des noyaux cellulaires composant les tissus constituants sur lesquels l'ultrason est projeté sur la base de l'information sur un degré de modulation.

6. Appareil d'analyse d'information de sujet (1, 1B, 1C) selon la revendication 1, comprenant en outre :

un moyen de discrimination (9) qui discrimine des propriétés de tissus constituants sur la base d'une information de caractéristique sur un rapport de composition des tissus constituants composant la région devant être examinée (18) calculée en utilisant l'information sur la fréquence spécifique extraite par le moyen d'extraction d'information (8a, 8b, 9a).

7. Appareil d'analyse d'information de sujet (1, 1B, 1C) selon la revendication 6, dans lequel le moyen de discrimination (9c) discrimine des propriétés des tissus constituants en comparant la valeur calculée de l'information de caractéristique avec un seuil calculé au préalable.

8. Appareil d'analyse d'information de sujet (1, 1B, 1C) selon la revendication 6, comprenant en outre :

un moyen de balayage (3) qui balaye la région devant être examinée (18) sur laquelle est projetée la lumière d'éclairage d'une fréquence prédéterminée par le moyen de projection de lumière (4, 5 ; 4b, 5a, 5b ; 4c, 5a, 5b) et également sur laquelle est projeté l'ultrason de la première fréquence par le moyen de projection d'ultrason (2) ; et
un moyen de traitement d'affichage (9d) qui affiche, comme une image, une information d'un résultat de discrimination de discrimination de propriétés de tissus dans des tissus constituants composant la région devant être examinée (18) de chaque position balayée.

9. Procédé d'analyse d'information de sujet comprenant :

une étape de projection de lumière d'un moyen de projection de lumière (4, 5 ; 4b, 5a, 5b ; 4c, 5a, 5b) projetant sur une région devant être examinée (18) dans un sujet (17) une lumière d'une deuxième fréquence ;
une étape de projection d'ultrason d'un moyen de projection d'ultrason (2) projetant sur le sujet (17) un ultrason d'une première fréquence ;

une étape de réception de lumière d'un moyen de réception de lumière (6, 7 ; 5d, 7) recevant une lumière modulée en fréquence comme une lumière diffusée correspondant à une lumière de projection de la deuxième fréquence à partir de la région devant être examinée (18) conformément à l'effet Doppler par vibration de tissus constituants composant la région devant être examinée (18) provoqué par l'ultrason d'une première fréquence ; et

une étape d'extraction d'information d'un moyen d'extraction d'information (8a, 8b, 9a) extrayant une information d'intensité sur une fréquence spécifique décalée d'un multiple entier de la première fréquence par rapport à la deuxième fréquence de la lumière modulée en fréquence reçue par l'étape de réception de lumière, **caractérisé en ce que** le procédé comprend en outre :

une étape de calcul de fraction volumique par un moyen de calcul de fraction volumique (9b) calculant, comme une information de caractéristique sur un rapport de composition des tissus constituants composant la région devant être examinée lorsque les tissus constituants sont composés de noyaux cellulaires et de cytoplasme, une valeur de calcul d'une fraction volumique indiquant une proportion volumique occupée par les noyaux cellulaires sur la base d'un degré de modulation calculé à l'étape d'extraction d'information ; et une étape d'affichage d'un moyen d'affichage (16) affichant un rapport noyaux cellulaires/cytoplasme calculé sur la base de la fraction volumique calculée à l'étape de calcul de fraction volumique, dans lequel l'étape d'extraction d'information calcule le degré de modulation de la lumière modulée en fréquence pour calculer la valeur de calcul d'une fraction volumique à partir d'une information d'intensité avec au moins deux fréquences spécifiques différentes décalées d'un multiple entier de la première fréquence par rapport à la deuxième fréquence.

**FIG.1**

# FIG.2

CELL OF 10 μm IN SIZE (THE SAME ALSO APPLIES TO 15 μm, 20 μm IN SIZE)

# FIG.3

CONTINUOUS LIGHT

HIGH FREQUENCY PULSE (ULTRASOUND PULSE)

ULTRASOUND IN REGION TO BE EXAMINED

TIMING PULSE

SIGNAL PROCESSING

SCANNING CONTROL SIGNAL

DRIVE SIGNAL

TIME

# FIG.4

```
                    ( START )
                        │
                        ▼
              ┌──────────────────┐
              │ INITIAL SETTING  │─ S1
              └──────────────────┘
                        │
                        ▼
              ┌──────────────────┐
              │  PARAMETER k=1   │─ S2
              └──────────────────┘
                        │
     ┌──────────────────┤
     │                  ▼
     │   ┌───────────────────────────────────┐
     │   │   RADIATE LIGHT ONTO SCANNING      │─ S3
     │   │ POSITION Rk AS REGION TO BE EXAMINED│
     │   └───────────────────────────────────┘
     │                  │
     │                  ▼
     │   ┌───────────────────────────────────┐
     │   │    RADIATE ULTRASOUND ONTO         │─ S4
     │   │      SCANNING POSITION Rk          │
     │   └───────────────────────────────────┘
     │                  │
     │                  ▼
     │   ┌───────────────────────────────────┐
     │   │  DETECT FREQUENCY-MODULATED        │─ S5
     │   │ SCATTERED LIGHT BY LIGHT DETECTOR  │
     │   └───────────────────────────────────┘
     │                  │
     │                  ▼
     │   ┌───────────────────────────────────┐
     │   │  CONVERT TIME-SERIES INTENSITY     │─ S6
S13  │   │  DATA I(t) TO FREQUENCY DATA I(f)  │
     │   └───────────────────────────────────┘
┌──────────────┐       │
│ SCANNING     │       ▼
│ DRIVER MOVES │   ┌──────────────────┐
│ SCANNING     │   │ CALCULATE DEGREE │─ S7
│ SECTION BY   │   │ OF MODULATION m  │
│ ONE STEP IN  │   └──────────────────┘
│ HORIZONTAL   │       │
│ DIRECTION    │       ▼
└──────────────┘   ┌──────────────────────┐
     │             │ CALCULATE MAXIMUM    │─ S8
     │             │ VALUE vmax OF        │
     │             │ VIBRATION SPEED      │
     │             └──────────────────────┘
     │                 │
     │                 ▼
     │             ┌──────────────────────┐
     │             │ CALCULATE VOLUME     │─ S9
     │             │ FRACTION Xp AND N/C  │
     │             │ RATIO                │
     │             └──────────────────────┘
     │                 │
     │                 ▼
     │             ┌──────────────────────┐
     │             │ SAVE VOLUME FRACTION │─ S10
     │             │ Xp AND N/C RATIO IN  │
     │             │ MEMORY               │
     │             └──────────────────────┘
     │  ┌─────────┐    │
     │  │ k=k+1   │─S12 ▼      S11
     │  └─────────┘   ╱─────────────╲
     └───NO──────────│ SCANNING END? │
                     ╲─────────────╱
                           │ YES
                           ▼
            ┌───────────────────────────────┐
            │ DISPLAY IMAGE OF N/C RATIO     │─ S14
            │ (OR VOLUME FRACTION Xp)        │
            │ TOGETHER WITH DISCRIMINATION   │
            │ INFORMATION                    │
            └───────────────────────────────┘
                           │
                           ▼
                       ( END )
```

# FIG.5

EP 2 378 272 B1

# FIG.6

START

INITIAL SETTING —S1

PARAMETER k=1 —S2

RADIATE LIGHT ONTO SCANNING
POSITION $R_k$ AS REGION TO BE EXAMINED —S3

RADIATE ULTRASOUND ONTO
SCANNING POSITION $R_k$ —S4

CAUSE FREQUENCY-MODULATED SCATTERED
LIGHT TO INTERFERE WITH REFERENCE
LIGHT AND DETECT INTERFERENCE
LIGHT BY LIGHT DETECTOR —S25

CONVERT TIME-SERIES INTENSITY
DATA I(t) TO FREQUENCY DATA I(f) —S6

S13

SCANNING DRIVER MOVES
SCANNING SECTION BY ONE
STEP IN HORIZONTAL DIRECTION

CALCULATE DEGREE OF
MODULATION m —S7

CALCULATE MAXIMUM VALUE
$v_{max}$ of VIBRATION SPEED —S8

CALCULATE VOLUME
FRACTION $X_p$ AND N/C RATIO —S9

SAVE VOLUME FRACTION
$X_p$ AND N/C RATIO IN MEMORY —S10

S12

k=k+1

NO

S11

SCANNING END?

YES

DISPLAY IMAGE OF N/C RATIO
(OR VOLUME FRACTION $X_p$) TOGETHER
WITH DISCRIMINATION INFORMATION —S14

END

# FIG.7

EP 2 378 272 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2008170363 A
- EP 1810610 A1
- US 6041248 A
- WO 03050249 A2
- JP 2088743 A

### Non-patent literature cited in the description

- **MAHAN G D et al.** ultrasonic tagging of light: theory. *proceedings of the national academy of sciences,* 01 January 1998, vol. 95, 14015-14019